(19) Europäisches Patentamt / European Patent Office / Office européen des brevets

(11) **EP 3 884 839 B1**

(12) **EUROPEAN PATENT SPECIFICATION**

(45) Date of publication and mention
of the grant of the patent:
**28.01.2026 Bulletin 2026/05**

(21) Application number: **19888012.2**

(22) Date of filing: **20.11.2019**

(51) International Patent Classification (IPC):
*A61B 1/00* (2006.01)    *A61L 31/14* (2006.01)
*A61L 31/04* (2006.01)    *A61B 1/015* (2006.01)

(52) Cooperative Patent Classification (CPC):
**A61L 31/042; A61L 31/14;** A61B 1/015

(86) International application number:
**PCT/JP2019/045384**

(87) International publication number:
**WO 2020/105668 (28.05.2020 Gazette 2020/22)**

(54) **ENDOSCOPE VISUAL FIELD-SECURING VISCOELASTIC COMPOSITION**

VISKOELASTISCHE ENDOSKOPSICHTFELDSCHUTZZUSAMMENSETZUNG

COMPOSITION VISCOÉLASTIQUE DE PROTECTION DE CHAMP VISUEL D'ENDOSCOPE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**ME**

(30) Priority: **22.11.2018 JP 2018218960**

(43) Date of publication of application:
**29.09.2021 Bulletin 2021/39**

(73) Proprietors:
• **Jichi Medical University
Chiyoda-ku, Tokyo 102-0093 (JP)**
• **Otsuka Pharmaceutical Factory, Inc.
Naruto-shi, Tokushima 772-8601 (JP)**

(72) Inventors:
• **YANO Tomonori
Shimotsuke-shi, Tochigi 329-0498 (JP)**
• **OHHATA Atsushi
Naruto-shi, Tokushima 772-8601 (JP)**
• **HIRAKI Yuji
Naruto-shi, Tokushima 772-8601 (JP)**

(74) Representative: **Santarelli
Tour Trinity
1 bis Place de la Défense
92400 Courbevoie (FR)**

(56) References cited:
**WO-A1-2017/057504**

## Description

## Technical Field

The present invention relates to a viscoelastic composition suitable for use in securing the field of view of an endoscope and is defined by the appended claims. More particularly, the invention relates to a viscoelastic composition that is suitable for use in securing the field of view of an endoscope when opaque dark-colored liquid or a semi-solid material accumulates inside a canal and obstructs the field of view of the endoscope, the viscoelastic composition securing the field of view by pushing away the liquid or semi-solid material, or for use in securing the field of view of an endoscope when the field of view of an endoscope is obstructed by sustained hemorrhage inside a canal.

## Background Art

Endoscopes may be used to observe the inside of a fine canal such as an alimentary canal or a bile duct. However, when dark-colored liquid that would block light, such as blood, intestinal juice or bile, or a semi-solid material such as food residue or excrement accumulates in the inside of a canal, or when sustained hemorrhage occurs inside a canal, the field of view of an endoscope is obstructed, and the inside state of the canal cannot be fully observed. Under such circumstances, the field of view of an endoscope has been hitherto secured by supplying water or gas into the inside of the canal. Supplying water means injecting water such as distilled water or tap water into the canal and washing away the liquid or the semi-solid material inside the canal. The field of view is temporarily secured in this method by removing the liquid or the semi-solid material inside the canal; however, in many cases, the liquid or the semi-solid material thus removed is mixed with the water, the suspended opaque liquid diffuses in the canal, and securing the field of view is still inadequate. On the other hand, supplying gas means blowing gas such as air or carbon dioxide into the inside of the canal thereby removing the liquid or the semi-solid material inside the canal. However, although the field of view is also temporarily secured in this method by removing the liquid or the semi-solid material inside the canal, when there is a hemorrhage site, removing blood is difficult, and the field of view is not secured. Therefore, there have been problems associated with difficulties in identifying a hemorrhage site and operating for a hemostasis treatment.

As countermeasures for such problems, it has been reported that the field of view is secured in urgent endoscopy by injecting, via a forceps opening, a transparent jelly beverage which can be easily injected through a forceps opening of an endoscope but has a viscosity of the extent that does not immediately mix with blood or residue, filling the lumen of the treatment site with the jelly, and then continuing to slowly injecting the jelly even during the insertion of a treatment tool without using supplying gas but by using BioShield Irrigator (US Endoscopy) as a forceps lid (Non-Patent Literatures 1 and 2). It has also been reported that the field of view of an endoscope is secured by using a viscoelastic composition containing a thickening substance and water, particularly the viscoelastic composition having a loss tangent (tan δ) of 0.6 or less (Patent Literature 1).

## Citation List

## Patent Literature

Patent Literature 1: WO 2017/057504 A

## Non-Patent Literature

Non-Patent Literature 1: YANO Tomonori, and 13 others, "Gel immersion endoscopy: New method for securing field of view in urgent endoscopy", Progress of Digestive Endoscopy, June 1, 2015, Vol. 87, Supplement, p. s85
Non-Patent Literature 2: YANO T, 9 others, "Gel immersion endoscopy: a novel method to secure the visual field during endoscopy in bleeding patients (with videos)", Gastrointestinal Endoscopy, April 2016, Vol. 83, No. 4, pp. 809-811

## Summary of Invention

## Technical Problem

An object of the present invention, which is defined by the appended claims, is to provide a viscoelastic composition suitable for use in securing the field of view of an endoscope. More particularly, an object of the invention is to provide a viscoelastic composition suitable for use in securing the field of view of an endoscope when opaque dark-colored liquid or a semi-solid material accumulates inside a canal and obstructs the field of view of the endoscope, the viscoelastic composition securing the field of view by pushing away the liquid or the semi-solid material, or for use in securing the field of view of an endoscope when the field of view of the endoscope is obstructed by sustained hemorrhage inside a canal, and to provide a method for securing the field of view of an endoscope using the viscoelastic composition. Even more particularly, the inventors of the present invention have found that when a composition merely having high viscosity is used as a composition for securing the field of view of an endoscope, it is possible to temporarily push away liquid or a semi-solid material aside, but the liquid or semi-solid material that has been physically pushed away for the moment diffuses or suspends returning into the field of view in a short period of time, and consequently, it becomes difficult to secure the desired field of view. The inventors have also found that when a composition merely having high viscosity is used in the situation where sustained hemorrhage occurs within the field of view of the endoscope or in the periphery of the field of view and the blood is discharged into the inside of the composition filling the lumen, the blood diffuses or suspends inside the composition in a short period of time and, consequently, it becomes difficult to secure the desired field of view. Thus, an object of the present invention is to provide a viscoelastic composition that does not have such defects for securing the field of view of an endoscope using the viscoelastic composition.

**Solution to Problem**

The inventors of the present invention conducted intensive studies in order to solve the aforementioned problems and, as a result, the inventors surprisingly found that the use of a composition having a shear storage modulus G' of 0.7 Pa or more makes it possible to obtain a viscoelastic composition suitable for use in securing the field of view of an endoscope regardless of its loss tangent (tan $\delta$), and a method for securing the field of view of an endoscope using the viscoelastic composition. Thus, the inventors finally completed the present invention as described below.

[1] A viscoelastic composition for securing the field of view of an endoscope, the viscoelastic composition comprising a thickening substance and water and having a shear storage modulus G' of 0.7 Pa or more, wherein the viscoelastic composition is colorless and transparent, wherein the shear storage modulus G' is measured under the measurement conditions as follows: measurement temperature: 25.0°C, gap: 1.000 mm, stress: 1,000 mPa, and frequency: 0.5000 Hz, and the shear storage modulus G' is measured as a value obtained after 30 minutes from the initiation of measurement, and wherein the viscoelastic composition has a loss tangent of more than 0.6, wherein the loss tangent is measured under the measurement conditions as follows: measurement temperature: 25.0°C, gap: 1.000 mm, stress: 1,000 mPa, and frequency: 0.5000 Hz, and the loss tangent is measured as a value obtained after 30 minutes from the initiation of measurement, and wherein the thickening substance consists in a combination of a polysaccharide having, as the main chain, a structure in which glucose is glycoside-bonded and a polysaccharide having, as the main chain, a structure in which mannose is glycoside-bonded, and the weight ratio of the polysaccharide having, as the main chain, a structure in which glucose is glycoside-bonded to polysaccharide having, as the main chain, a structure in which mannose is glycoside-bonded is 5:95 to 90:10.

[2] The viscoelastic composition according to [1], wherein the viscoelastic composition has a shear storage modulus G' of 7.0 Pa or less.

[3] An endoscope comprising a viscoelastic composition for securing the field of view of an endoscope, the viscoelastic composition comprising a thickening substance and water and having a shear storage modulus G' of 0.7 Pa or more, wherein the viscoelastic composition is colorless and transparent, wherein the shear storage modulus G' is measured under the measurement conditions as follows: measurement temperature: 25.0°C, gap: 1.000 mm, stress: 1,000 mPa, and frequency: 0.5000 Hz, and the shear storage modulus G' is measured as a value obtained after 30 minutes from the initiation of measurement, and wherein the viscoelastic composition has a loss tangent of more than 0.6, wherein the loss tangent is measured under the measurement conditions as follows: measurement temperature: 25.0°C, gap: 1.000 mm, stress: 1,000 mPa, and frequency: 0.5000 Hz, and the loss tangent is measured as a value obtained after 30 minutes from the initiation of measurement and wherein the thickening substance consists in a combination of a polysaccharide having, as the main chain, a structure in which glucose is glycoside-bonded and a polysaccharide having, as the main chain, a structure in which mannose is glycoside-bonded, and the weight ratio of the polysaccharide having, as the main chain, a structure in which glucose is glycoside-bonded to polysaccharide having, as the main chain, a structure in which mannose is glycoside-bonded is 5:95 to 90:10.

[4] The endoscope according to [3], wherein the viscoelastic composition has a shear storage modulus G' of 7.0 Pa or less.

**Advantageous Effects of Invention**

The viscoelastic composition of the present invention can be suitably used for use in securing the field of view of an endoscope. Specifically, the viscoelastic composition of the present invention is capable of physically pushing away liquid such as blood, intestinal juice or bile, or a semi-solid material such as food residue or excrement by means of an elastic component of the composition when injected into the inside of a canal via a channel of an endoscope. Further, since the viscoelastic composition of the present invention has a property of not easily mixing with the aforementioned liquid or semi-solid material due to an elastic component of the composition, the viscoelastic composition can prevent the liquid or semi-solid material that has been physically pushed away from diffusion or suspension returning into the field of view in a short period of time. Moreover, due to the property of being hardly miscible with the liquid or the semi-solid material, when sustained hemorrhage occurs within the field of view of an endoscope or in the periphery of the field of view and the blood is discharged into the inside of the viscoelastic composition filling the lumen, the viscoelastic composition of the present invention can prevent the blood from diffusing and suspending inside the composition in a short period of time, and consequently, the viscoelastic composition enables a clear field of view to be maintained over a relatively long period of time.

**Brief Description of Drawings**

Fig. 1 is a diagram showing a photograph of a proximal part of a medical endoscope.
Fig. 2A is a conceptual diagram illustrating the state of the inside of an alimentary canal that has hemorrhaged.
Fig. 2B is a conceptual diagram illustrating the state in which water has been injected into the hemorrhaged alimentary canal.
Fig. 3 is a diagram illustrating an example of a camera image obtainable when water is injected into an alimentary canal via a forceps opening of an endoscope.
Fig. 4A is a conceptual diagram illustrating the state of the inside of an alimentary canal that has hemorrhaged.
Fig. 4B is a conceptual diagram illustrating the state in which the viscoelastic composition of the present invention has been injected into the hemorrhaged alimentary canal.
Fig. 5 is a diagram illustrating an example of a camera image obtainable when the viscoelastic composition of the present invention is injected into an alimentary canal via a forceps opening of an endoscope.

**Description of Embodiments**

The viscoelastic composition of the present invention contains a thickening substance and water.
The term "thickening substance" of the present invention means a substance having an effect of increasing viscosity when dissolved or dispersed in water. The thickening substance of the present invention comprises a combination of two kinds of components, and is as defined in the claims. Examples of such a thickening substance include ,polysaccharides such as Aureobasidium culture solution, flaxseed gum, gum Arabic, arabinogalactan, alginic acid and salts thereof, propylene glycol alginate ester, welan gum, Cassia gum, gum ghatti, curdlan, carrageenan, karaya gum, xanthan gum, guar gum, guar gum enzymolysis products, psyllium seed gum, Artemisia sphaerocephala seed gum, gellan gum, succinoglycan, tamarind gum, tara gum, tragacanth gum, furcellaran, funoran, pullulan, pectin, macrophomopsis gum, Rhamsan gum, locust bean gum, starch grafted acrylate, acetylated distarch adipate, acetylated oxidized starch, acetylated distarch phosphate, starch sodium octenylsuccinate, carboxymethyl cellulose and salts thereof, carboxymethyl ethyl cellulose, starch acetate, oxidized starch, sodium starch glycolate, hydroxypropyl distarch phosphate, hydroxypropyl cellulose, hydroxypropyl starch, hydroxypropyl methyl cellulose, hydroxymethyl cellulose, hydroxyethyl methyl cellulose, hydroxyethyl cellulose, ethyl cellulose, methyl cellulose, cellulose, distarch phosphate, monostarch phosphate, phosphated distarch phosphate, fucoidan, diutan gum, glucomannan, hyaluronic acid and salts thereof, keratan sulfate, heparin, chondroitin sulfate, dermatan sulfate, scleroglucan, schizophyllan, okra extracts, Krantz aloe extracts, sesbania gum, agarose, agaropectin, amylose, amylopectin, pregelatinized starch, inulin, levan, graminan, agar, hydroxypropyl methylcellulose stearoxy ether, dextran, dextrin, croscarmellose sodium, glucuronoxylan, and arabinoxylan;. However, the examples are not limited to these.
The thickening substance of the present invention is a combination of a polysaccharide having, as the main chain, a structure in which glucose is glycoside-bonded (for example, xanthan gum or cellulose),and a polysaccharide having, as the main chain, a structure in which mannose is glycoside-bonded (for example, guar gum or locust bean gum).
The thickening substance of the present invention is preferably a substance that is highly safe when applied into the living body, that is, a substance that hardly or never has adverse effect on the human body. From this perspective, it is preferable that the thickening substance of the present invention is a thickening substance having at least product quality equal to or higher than the products recognized as food additives. From the viewpoint of safety in term of the absence of impurities, thickening substances such as hydrophilic polymers obtained by synthesis may be used.

When naturally occurring substances (for example, polysaccharides such as xanthan gum, locust bean gum, and guar gum) are used as the thickening substance of the present invention, since the physical properties vary significantly depending on the difference in manufacturers or lots, it is preferable to prepare the viscoelastic composition of the present invention by changing the concentration (for example, % by weight) by adding water, which is a dispersing medium, or the like while checking the desired physical properties including shear storage modulus.

When a combination of two kinds of components is used as the thickening substance of the present invention, a viscoelastic composition having desired physical properties may be appropriately obtained by changing the mixing ratio of the two or more kinds of components. Examples of the polysaccharide having, as the main chain, a structure in which glucose is glycoside-bonded include xanthan gum and cellulose, and the mixing ratio of the polysaccharide having, as the main chain, a structure in which glucose is glycoside-bonded (% by weight of the polysaccharide in the entirety of the thickening substance) is preferably 5% to 90% by weight, more preferably 10% to 80% by weight, and even more preferably 20% to 70% by weight. Examples of the polysaccharide having, as the main chain, a structure in which mannose is glycoside-bonded include guar gum and locust bean gum, and the mixing ratio of the polysaccharide having, as the main chain, a structure in which mannose is glycoside-bonded (% by weight of the polysaccharide in the entirety of the thickening substance) is preferably 10% to 90% by weight, more preferably 20% to 85% by weight, and even more preferably 30% to 80% by weight.

In the present invention the thickening substance consists in a combination of a polysaccharide having, as the main chain, a structure in which glucose is glycoside-bonded and a polysaccharide having, as the main chain, a structure in which mannose is glycoside-bonded, and for example, a combination of xanthan gum and guar gum or a combination of xanthan gum and locust bean gum. The weight ratio of the combination may be appropriately determined while checking whether desired physical property values including shear storage modulus are obtained or not; however, the weight ratio of the polysaccharide having, as the main chain, a structure in which glucose is glycoside-bonded to polysaccharide having, as the main chain, a structure in which mannose is glycoside-bonded is 5 : 95 to 90 : 10, preferably 10 : 90 to 80 : 20, and more preferably 20 : 80 to 70 : 30. Also, when a combination of two kinds of components is used as the thickening substance of the present invention, as described above, it is preferable to prepare the viscoelastic composition of the present invention via changing the concentration (for example, % by weight) by adding water, which is a dispersing medium, while checking desired physical property values including shear storage modulus or the like.

The "water" used in the viscoelastic composition of the present invention is not particularly limited; however, examples of the water include soft water, pure water, ultrapure water, deionized water, distilled water, and tap water. The water may also be a physiological aqueous solution such as physiological saline, Ringer's solution, or acetate Ringer's solution.

The viscoelastic composition of the present invention has a shear storage modulus G' of 0.7 Pa or more. In an embodiment of the present invention, the shear storage modulus G' of the viscoelastic composition of the present invention is defined as a value measured at room temperature, particularly 25°C. The viscoelastic composition of the present invention is provided for use in securing the field of view of an endoscope. According to a typical embodiment in such use, the viscoelastic composition is delivered from a proximal part of an endoscope, through a forceps channel, to a distal part of the endoscope and then discharged therefrom into a canal, so that liquid such as blood, intestinal juice or bile, or a semi-solid material such as food residue or excrement are physically pushed away over a certain period of time. In such an embodiment, the time taken by the viscoelastic composition of the present invention to be delivered from the proximal part to the distal part of an endoscope and then act in the body is usually as short as about several minutes, and the temperature of the viscoelastic composition, which is usually stored and injected at room temperature (particularly 25°C), would not change significantly at the time of operation of the viscoelastic composition. Further, if the retention time of the viscoelastic composition of the present invention inside a canal is lengthened by some reasons, the temperature of the viscoelastic composition may rise, the physical property values may change, and consequently securing the desired field of view may become difficult. However, even in such cases, by additionally injecting the viscoelastic composition of the present invention that has been stored at room temperature (particularly 25°C), the temperature of the viscoelastic composition in the vicinity of the distal part of an endoscope, which is an area of observation, may always be kept at near room temperature (particularly 25°C). Therefore, it is reasonable to define the shear storage modulus G' of the viscoelastic composition of the present invention as a value measured at room temperature, particularly at 25°C.

The shear storage modulus G' may be measured using any rheometer, for example, HAAKE MARSIII manufactured by Thermo Fisher Scientific Inc. According to the present invention, the shear storage modulus G' of the viscoelastic composition of the present invention is measured under the measurement conditions as follows: measurement temperature: 25.0°C, gap: 1.000 mm, stress: 1,000 mPa, and frequency: 0.5000 Hz, and the shear storage modulus G' is measured as a value obtained after 30 minutes from the initiation of measurement.

In an embodiment of the present invention, the shear storage modulus G' of the viscoelastic composition of the present invention is 0.7 Pa or more, 0.75 Pa or more, 0.8 Pa or more, 0.85 Pa or more, 0.9 Pa or more, 0.95 Pa or more, 1.0 Pa or more, 1.05 Pa or more, 1.1 Pa or more, 1.15 Pa or more, 1.2 Pa or more, 1.25 Pa or more, 1.3 Pa or more, 1.35 Pa or more, 1.4 Pa or more, 1.45 Pa or more, 1.5 Pa or more, 1.55 Pa or more, 1.6 Pa or more, 1.65 Pa or more, 1.7 Pa or more, 1.75 Pa or more, 1.8 Pa or more, 1.85 Pa or more, 1.9 Pa or more, 1.95 Pa or more, 2.0 Pa or more, 2.1 Pa or more, 2.2 Pa or more, 2.3 Pa or more, 2.4 Pa or more, 2.5 Pa or more, 2.6 Pa or more, 2.7 Pa or more, 2.8 Pa or more, 2.9 Pa or more, 3.0 Pa or more, 3.1 Pa or more, 3.2 Pa or more, 3.3 Pa or more, 3.4 Pa or more, or 3.5 Pa or more.

In an embodiment of the present invention, the shear storage modulus G' of the viscoelastic composition of the present invention is 7.0 Pa or less, 6.5 Pa or less, 6.0 Pa or less, 5.5 Pa or less, 5.0 Pa or less, 4.5 Pa or less, 4.0 Pa or less, 3.5 Pa or less, or 3.0 Pa or less.

In an embodiment of the present invention, the shear storage modulus G' of the viscoelastic composition of the present invention is 0.7 to 7.0 Pa, 0.7 to 6.5 Pa, 0.7 to 6.0 Pa, 0.7 to 5.5 Pa, 0.7 to 5.0 Pa, 0.7 to 4.5 Pa, 0.7 to 4.0 Pa, 0.7 to 3.5 Pa, or 0.7 to 3.0 Pa, and in another embodiment of the present invention, the shear storage modulus G' of the viscoelastic composition of the present invention is 0.75 to 7.0 Pa, 0.75 to 6.5 Pa, 0.75 to 6.0 Pa, 0.75 to 5.5 Pa, 0.75 to 5.0 Pa, 0.75 to 4.5 Pa, 0.75 to 4.0 Pa, 0.75 to 3.5 Pa, or 0.75 to 3.0 Pa. In still another embodiment of the present invention, the shear storage modulus G' of the viscoelastic composition of the present invention is 0.8 to 7.0 Pa, 0.8 to 6.5 Pa, 0.8 to 6.0 Pa, 0.8 to 5.5 Pa, 0.8 to 5.0 Pa, 0.8 to 4.5 Pa, 0.8 to 4.0 Pa, 0.8 to 3.5 Pa, or 0.8 to 3.0 Pa.

When injected into the inside of a canal via a channel of an endoscope, the viscoelastic composition having a shear storage modulus G' of 0.7 Pa or more can physically push away liquid such as blood, intestinal juice or bile, or a semi-solid material such as food residue or excrement by an elastic component of the composition. Further, since the viscoelastic composition exhibits a property of being not easily miscible with the liquid or the semi-solid material due to the elastic component, the viscoelastic composition can prevent the liquid or semi-solid material that has been physically pushed away from diffusion or suspension returning into the field of view in a short period of time, or when sustained hemorrhage occurs within the field of view of the endoscope or in the periphery of the field of view and the blood is discharged into the inside of the composition filling the lumen, the viscoelastic composition can prevent the blood from diffusing and suspending inside the composition in a short period of time. As a result, the viscoelastic composition makes it possible to maintain a clear field of view over a relatively long period of time. As the shear storage modulus G' of the viscoelastic composition of the present invention becomes higher exceeding 0.7 Pa, the property of physically pushing away the liquid or the semi-solid material, or the property of not easily mixing with the liquid or the semi-solid material becomes more notable. On the other hand, when the shear storage modulus G' of the viscoelastic composition is smaller than 0.7 Pa, it becomes difficult for the viscoelastic composition to physically push away the liquid or the semi-solid material inside a canal, and even if the viscoelastic composition could push it away, since the viscoelastic composition is easily miscible with the liquid or the semi-solid material, the liquid or the semi-solid material returns into the field of view in a short period of time by diffusion or suspension. Further, when sustained hemorrhage occurs within the field of view of the endoscope or in the periphery of the field of view and the blood is discharged into the inside of the composition filling the lumen, the blood diffuses and suspends inside the composition in a short period of time and therefore, consequently, it would be impossible to maintain a clear field of view. Besides, when the viscoelastic composition of the present invention is injected into an application site via a forceps channel (inner diameter about 2.0 to 3.8 mm), particularly when the viscoelastic composition is injected into an application site via a forceps channel in a state in which a treatment tool such as forceps has been inserted, it can be said that a viscoelastic composition having a lower shear storage modulus G' is preferred from the viewpoint of making the channel passability of the viscoelastic composition better. From this point of view, the shear storage modulus G' of the viscoelastic composition of the present invention is preferably 7.0 Pa or less, more preferably 6.5 Pa or less, and even more preferably 6.0 Pa or less. Upon considering these, it is preferable to select the optimal shear storage modulus G' depending on the purpose of the viscoelastic composition of the present invention, the application site where the viscoelastic composition of the present invention is used with an endoscope, or the like.

In an embodiment of the present invention, the viscoelastic composition of the present invention has a loss tangent (tan δ) of higher than 0.6. The "loss tangent" is defined as a value obtained by dividing the shear loss modulus G" by the shear storage modulus G' as shown in the following formula.

[Chemical Formula 1]

$$\tan \delta = G'' / G'$$

In an embodiment of the present invention, the loss tangent of the viscoelastic composition of the present invention is defined as a value measured at room temperature, particularly at 25°C.

The loss tangent may be measured using any rheometer, for example, HAAKE MARSIII manufactured by Thermo Fisher Scientific Inc. The loss tangent of the viscoelastic composition of the present invention is measured under the measurement conditions as follows: measurement temperature: 25.0°C, gap: 1.000 mm, stress: 1,000 mPa, and frequency: 0.5000 Hz, and the loss tangent is measured as a value obtained after 30 minutes from the initiation of measurement.

In an embodiment of the present invention, the loss tangent of the viscoelastic composition of the present invention is 3.0 or less, 2.9 or less, 2.8 or less, 2.7 or less, 2.6 or less, 2.5 or less, 2.4 or less, 2.3 or less, 2.2 or less, 2.1 or less, 2.0 or less, 1.9 or less, 1.8 or less, 1.7 or less, 1.6 or less, 1.5 or less, 1.4 or less, 1.3 or less, 1.2 or less, 1.1 or less, 1.0 or less, 0.95 or less, 0.9 or less, 0.85 or less, 0.8 or less, 0.75 or less, 0.7 or less, or 0.65 or less.

In an embodiment of the present invention, the loss tangent of the viscoelastic composition of the present invention is 0.65 or more, 0.7 or more, 0.75 or more, 0.8 or more, 0.85 or more, 0.9 or more, 0.95 or more, or 1.0 or more.

The viscoelastic composition of the present invention may contain additional components such as a dispersant for dispersing a thickening substance in water and an osmotic pressure regulator. The dispersant makes it difficult for aggregates of the thickening substance to be produced when the thickening substance is dispersed in water, and/or enables shortening of the time required for dispersing the thickening substance in water. The osmotic pressure regulator is to regulate the osmotic pressure of the viscoelastic composition of the present invention, and preferably, the osmotic pressure regulator enables adjusting the osmotic pressure of the viscoelastic composition of the present invention to an extent that is approximately the same as that of physiological saline (for example, as an osmotic pressure ratio with physiological saline, 0.6 to 1.8, preferably 0.7 to 1.5, and more preferably 0.8 to 1.2). Any substances having the above-described action may be used as the dispersant or the osmotic pressure regulator of the present invention, and a single substance (for example, glycerin) working as both dispersant and osmotic pressure regulator of the present invention may be used.

Furthermore, the viscoelastic composition of the present invention may contain additives such as a preservative and an antiseptic agent.

As described in WO 2017/057504 A (Patent Literature 1), the channel passability at the time when the viscoelastic composition is injected into an application site via a channel (for example, a forceps channel) of an endoscope can be increased by setting the hardness and viscosity of the viscoelastic composition to predetermined values. From such a viewpoint, in an embodiment of the present invention, the viscoelastic composition of the present invention may have a hardness of 550 N/m$^2$ or less, and preferably 400 N/m$^2$ or less, and/or may have a viscosity of 200 to 2,000 mPa·s, and preferably 500 to 1,500 mPa·s. In another embodiment of the present invention, the viscoelastic composition of the present invention may have a hardness of 550 N/m$^2$ or less, and preferably 400 N/m$^2$ or less, at room temperature, particularly at 25°C, and/or may have a viscosity of 200 to 2,000 mPa·s, and preferably 500 to 1,500 mPa·s, at room temperature, particularly at 25°C.

Hardness may be measured using any creepmeter. For example, hardness may be measured using a creepmeter Model RE2-33005C manufactured by YAMADEN Co., Ltd. as follows: a stainless steel Petri dish (outer diameter: 45 mm, inner diameter: 41 mm, external dimension: 18 mm, and internal dimension: 15 mm) is filled with a specimen that has been temperature-controlled to 25°C, the height of the specimen is aligned with the height of the Petri dish, the surface of the specimen is made flat, and measurement is carried out using a plunger (YAMADEN Co., Ltd., shape: disc, model: P-56, remarks: φ20 × t 8) under the conditions as follows: storage pitch: 0.02 sec, measurement distortion rate: 66.67%, measurement speed: 10 mm/sec, return distance: 5.00 mm, sample thickness: 15.00 mm, contact surface: diameter 20.00 mm, and contact area: 0.000 mm$^2$.

Viscosity may be measured using any rheometer. For example, viscosity may be determined using a rheometer HAAKE MARSIII manufactured by Thermo Fisher Scientific Inc., by mounting about 1 mL of a specimen on a lower plate (Unltere Plate TMP35), starting measurements using a sensor (Platte P35 Til) under the conditions as follows: measurement temperature: 25.0°C, gap: 1.000 mm, stress: 1,000 mPa, and frequency: 0.5000 Hz, and obtaining a value after 30 minutes from the initiation of the measurements.

Further, as described in WO 2017/057504 A (Patent Literature 1), a viscoelastic composition suitable for treatment under an endoscope (particularly, electrical treatment such as electroscission or electrocoagulation) may be obtained by setting the electrical conductivity of the viscoelastic composition to a predetermined value. From such a viewpoint, in an embodiment of the present invention, the viscoelastic composition of the present invention may have an electrical conductivity of 250 μS/cm or less, and preferably 200 μS/cm or less. In another embodiment of the present invention, the viscoelastic composition of the present invention may have an electrical conductivity of 250 μS/cm or less, and preferably 200 μS/cm or less, at room temperature, particularly at 25°C.

Electrical conductivity may be measured using any electrical conductivity meter. For example, electrical conductivity may be measured using an electrical conductivity meter CM-41X and a CT-57101C cell for low electrical conductivity measurement manufactured by DKK-TOA CORPORATION at measurement temperature: 25°C.

Since the viscoelastic composition of the present invention is to be applied into the body of an animal (human being), the viscoelastic composition is preferably sterilized before application. For example, the viscoelastic composition of the present invention is preferably sterilized under the conditions of 121°C for 15 minutes (Fo value control: Fo = 20). Since the viscoelastic composition may undergo changes in the physicochemical properties due to high temperature, in the case of performing a sterilization treatment, it is preferable to design a material so as to achieve intended physical properties after the sterilization treatment, or to select a material that undergoes less change in the physicochemical properties due to high temperature.

An object of the present invention is to provide a viscoelastic composition suitable for use in securing the field of view of an endoscope, and more particularly, the object is to provide a viscoelastic composition suitable for use in securing the field of view of an endoscope when opaque dark-colored liquid or a semi-solid material accumulates inside a canal and obstructs the field of view of the endoscope, the viscoelastic composition securing the field of view by pushing away the liquid or the semi-solid material, or for use in securing the field of view of an endoscope when the field of view of the endoscope is obstructed by sustained hemorrhage inside a canal, and to provide a method for securing the field of view of an endoscope using the viscoelastic composition. Meanwhile, the reasons why the field of view of an endoscope is obstructed are thought to be because a liquid such as blood, intestinal juice or bile, or a semi-solid material such as food residue or excrement: 1) accumulates in the lumen; 2) mixes with water to have impaired transparency; and 3) flows or diffuses due to supplying water. In order to satisfactorily secure the field of view, a transparent composition having viscoelasticity, which is different from the viscoelasticity of the liquid or the semi-solid material, can be injected into the lumen thereby physically pushing these away and removing these to make a space secured by the transparent composition. At the same time, the transparent composition can prevent itself from rapidly mixed with the liquid or the semi-solid material impairing its transparency and can suppress flow or diffusion of the liquid or the semi-solid material.

The viscoelastic composition of the present invention may be obtained by mixing a thickening substance with water, and specifically, the viscoelastic composition may be obtained by combining two or more kinds of the thickening substances or dissolving one kind of thickening substance in water or the like, subjecting the resultant to a heating treatment, and thereby imparting elasticity, or the like. If there are bubbles in the viscoelastic composition of the present invention, the field of view is obstructed. It is therefore preferable that the viscoelastic composition substantially has no bubbles. The phrase "substantially has no bubbles" as used herein means that there are no bubbles of a size or an amount to the extent that they make observation or operation within the field of view of an endoscope substantially impossible. For example, the phrase implies that bubbles cannot be recognized when the viscoelastic composition is observed with naked eyes. Further, for accurate observation within the field of view of an endoscope, it is preferable that the viscoelastic composition of the present invention is colorless and transparent. The phrase "colorless and transparent" as used here means that the viscoelastic composition is not colored and not opaque to the extent that makes observation or operation in the field of view of an endoscope substantially impossible. For example, the phrase means that the absorbance at an optical path length of 10 mm in the visible light wavelength range of 400 to 800 nm is 80% or lower.

The viscoelastic composition of the present invention is a viscoelastic composition for securing the field of view of an endoscope, and the "endoscope" includes endoscopes for all uses. In an embodiment of the present invention, the endoscope is a medical endoscope.

The method for securing the field of view of an endoscope comprises feeding the viscoelastic composition of the present invention from a proximal part of an endoscope, through a channel, to a distal part of the endoscope. In particular, the method for securing the field of view of an endoscope of the present invention comprises feeding the viscoelastic composition of the present invention from the proximal part of an endoscope, through a forceps channel (inner diameter about 2.0 to 3.8 mm), to the distal part of the endoscope, and particularly, the method comprises feeding the viscoelastic composition, through a forceps channel in a state in which a treatment tool such as forceps has been inserted, to the distal part of the endoscope.

With regard to the method for securing the field of view of an endoscope of the present invention, the "endoscope" includes endoscopes for all uses. In an embodiment of the present invention, the endoscope is a medical endoscope.

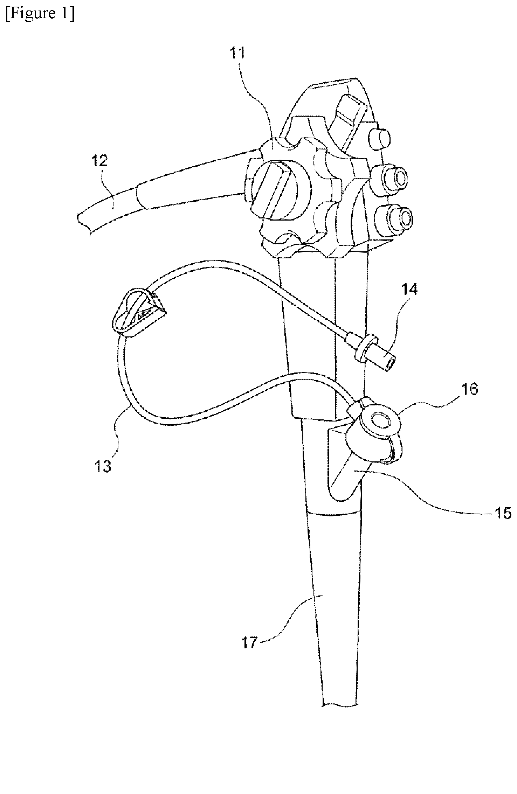

Fig. 1 is a diagram showing a photograph of a proximal part of a medical endoscope as a representative example of endoscopes. The proximal part of the medical endoscope includes a dial 11 for performing an angle manipulation; a scope connector unit 12 for transmitting light from a light source apparatus and transmitting the information of images to an electronic endoscope processor; and a forceps opening 15 through which a treatment tool such as forceps is inserted and conveyed to the distal part 1. A forceps channel that connects through from the forceps opening to the distal part, a water-supplying duct for washing the lens in the distal part with water, optical system, and the like are provided inside an endoscope flexible tube 17. A forceps lid 16 is provided at the forceps opening 15 of the medical endoscope illustrated in Fig. 1. One end of a tube 13 is connected to the forceps lid 16, and the other end of the tube is connected to a connector 14 which is to be mounted to a syringe or the like. The forceps lid 16 is provided inside with a valve body, one end of the tube 13 is opened toward a wall surface of the valve body inside the forceps lid 16, the wall surface being on the side of the forceps opening 15, so that even when a treatment tool such as forceps is inserted, the liquid that has been injected through the tube is prevented from flowing out due to the valve body (see, for example, JP 2014-155677 A).

**Examples**

**Reference Examples**

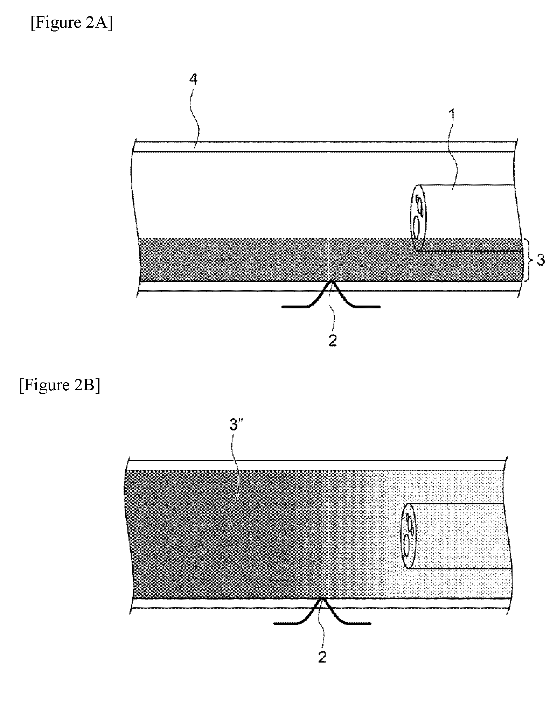

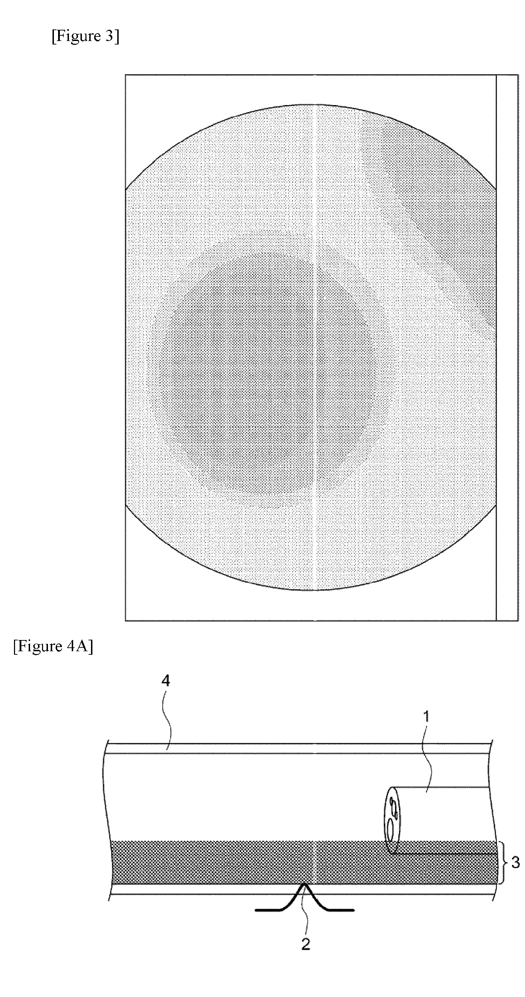

An endoscope may be inserted into, for example, an alimentary canal such as the rectum, thereby making it possible to observe the inside of the alimentary canal. When hemorrhage occurs in an alimentary canal 4, blood 3 accumulates inside the alimentary canal, and a hemorrhage site 2 cannot be observed (Fig. 2A). In such circumstances, the accumulated blood was attempted to be washed away with water by filling a syringe with tap water and injecting the water with the syringe through a tube 13, a forceps opening 15, and a forceps channel to the opening of the endoscope distal part 1, through which the water was injected into the alimentary canal. However, the injected water was mixed and suspended with the accumulated blood, and thus the water became turbid water 3". When the turbid water 3" accumulates inside the alimentary canal, the field of view of the endoscope is obstructed by the turbid water 3", and thus the hemorrhage site 2 cannot be observed. An operation cannot be continued accordingly (Fig. 2B and Fig. 3).

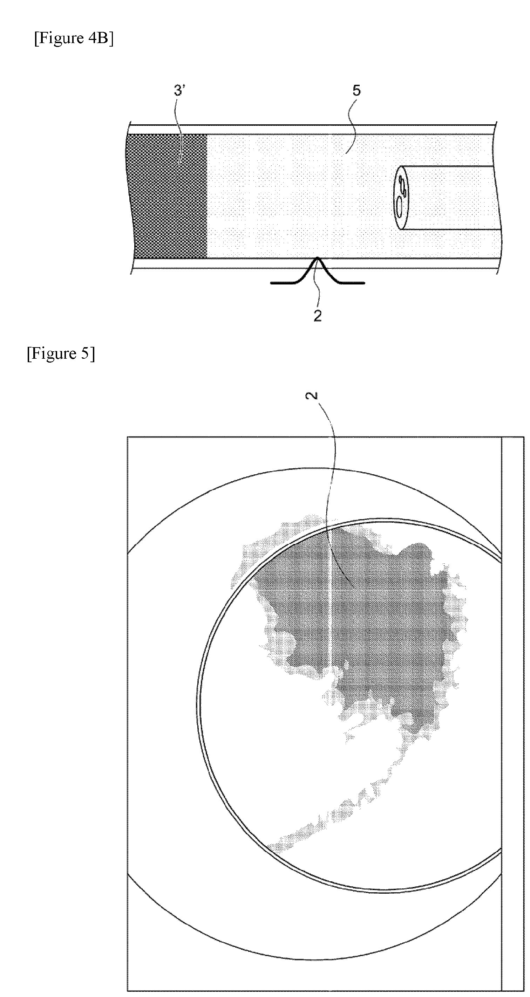

In contrast, the viscoelastic composition of the present invention makes it possible, when there is a hemorrhage site 2 in an alimentary canal 4, to push away the blood 3 that has accumulated inside the alimentary canal (blood 3') by injecting the viscoelastic composition from the distal part of the endoscope as illustrated in Fig. 4A and Fig. 4B. Also, as illustrated in Fig. 5, since the blood and the viscoelastic composition do not mix, the boundary can be clearly recognized and, consequently, it is possible to observe the hemorrhage site 2.

## Examples and Comparative Examples

In the present Examples or Comparative Examples, the relation between the viscoelastic properties (particularly, shear storage modulus G' and loss tangent tan $\delta$) of viscoelastic compositions used for endoscopy and the securement of the field of view in operating an endoscope was investigated. Examples which fall outside the scope of the claims are not part of the invention.

How to prepare viscoelastic composition specimens, how to measure physical property values related to viscoelastic properties, and how to evaluate viscoelastic compositions from the viewpoint of securing the field of view when the viscoelastic compositions are used for endoscopy are shown below.

(1) How to prepare specimens

While glycerin in an amount that would give a final concentration of 2.4% by weight was stirred at 1,400 rpm using a DISPER (PRIMIX Corporation) mounted to T.K. ROBOMICS No.071093 (PRIMIX Corporation), xanthan gum (XG: KELTROL CG-T, Lot; 7B5905K, manufacturer; Sansho Co., Ltd.) and locust bean gum (LBG: GENUGUM, Lot; SK71280, manufacturer; Sansho Co., Ltd.) as raw materials were introduced at a weight ratio at which the weight ratio of XG/LBG would be 2/8, and the mixture was dispersed for 2 minutes. The dispersion liquid was introduced into water that had been heated to 70°C, the container containing the dispersion liquid was washed, the water used for washing being combined with the dispersion liquid, and then the mixture was stirred for 5 minutes at 5,000 rpm using a DISPER. After stirring, water was appropriately added thereto to perform weight alignment, the mixture was stirred for 2 minutes at 3,000 rpm using a DISPER, and thereby various XG/LBG (2/8) specimens having intended concentrations (% by weight) were prepared.

Further, XG/LBG (5/5) specimens, XG/LBG (6/4) specimens, and XG/LBG (8/2) specimens having intended concentrations (% by weight) were prepared by methods similar to the above-described method, except that the weight ratio of XG/LBG was adjusted to 5/5, 6/4, and 8/2.

Moreover, various XG/GG (7/3) specimens having intended concentrations (% by weight) were prepared by methods similar to the above-described method, except that xanthan gum (XG: BISTOP D-3000C, Lot; 160523-01, manufacturer; San-Ei Gen F.F.I., Inc.) and guar gum (GG: RG100, Lot; 7091521, manufacturer; San-Ei Gen F.F.I., Inc.) as raw materials were introduced at a weight ratio at which the weight ratio of XG/GG would be 7/3.

Each of the specimens was sterilized under the conditions of 121°C for 15 minutes (Fo value control: Fo = 20) and then was used for the following various tests.

(2) How to measure viscoelastic properties (G', G", and tan $\delta$)

Viscoelastic properties were measured using a rheometer HAAKE MARSIII (Thermo Fisher Scientific Inc.). The inside of the laboratory was temperature-controlled to 25°C, subsequently a viscoelastic composition specimen (about 1 mL) that had been temperature-controlled to 25°C was mounted on a lower plate (Unltere Plate TMP35) and measured (measurement conditions: measurement temperature: 25.0°C, gap: 1.000 mm, stress: 1,000 mPa, and frequency: 0.5000 Hz) using a sensor (Platte P35 Til), and a value after 30 minutes from the initiation of measurement was determined.

(3) How to evaluate specimens in securing the field of view

As a method for evaluation in securing the field of view, a vinyl tube method, which will be described below, was used. This method simulates an actual environment for applying endoscopy, by using a flexible transparent plastic tube, such as a packaging bag made of thin polyethylene, to simulate an alimentary canal, and using the tip of a SURFRO indwelling needle inserted from the outer side toward the inner side of the plastic tube, to simulate a hemorrhage site inside an alimentary canal. In a laboratory set at room temperature of 23°C, the tip of a SURFRO indwelling needle was pierced from the outer side toward the inner side of a plastic tube (ELPA, packaging bag of MH-CT11H) having a width in a flat state of about 4.2 cm (accordingly, the diameter of a cross-sectional area in a state in which the inside is fully filled with liquid was about 2.6 to 2.7 cm) such that the needle would be parallel to the longitudinal axis direction of the plastic tube, and the indwelling needle was fixed to the plastic tube. Subsequently, the other end of the indwelling needle was connected to an extension tube X2-100 (TOP Corporation), and the distal portion of a flexible tube of an endoscope GIF-Q260J (Olympus Corporation) was inserted into the plastic tube. The SURFRO indwelling needle was arranged such that the tip of the needle would face the direction of insertion of the distal portion of the flexible tube of the endoscope, and the distance between the tip of the SURFRO indwelling needle and the distal portion of the flexible tube of the endoscope was set to approximately 3 to 6 cm. Subsequently, fixing tools were placed underneath two ends of the vicinity of the central portion of the plastic tube (a part extending for about 10 cm and including a site where the tip of the indwelling needle was fixed and a site where the distal portion of the flexible tube of the endoscope was inserted), and thereby the plastic tube was inclined such that initially introduced blood would stay in the vicinity of the central portion. Subsequently, 50 mL of pig blood was injected into the plastic tube, and after confirming that the blood accumulated in the vicinity of the central portion of the plastic tube, the extension tube connected to the indwelling needle was connected to a silicon tube L/S14 (Cole-Parmer Instrument Co.) connected to a pump MasterFlex L/S Model 07522-30 (Cole-Parmer Instrument Co.). Pig blood was continuously injected into the plastic tube using the pump via the silicon tube, extension tube, and indwelling needle (pump setting: periodical liquid supply mode, flow rate: 10 mL/min, on/off: 1 second/1 second, number of batches: 9999). This continuous injection of pig blood into the plastic tube simulates hemorrhage inside a canal such as an alimentary canal; however, a stricter simulation environment was set up by making the injection speed faster than generally assumed hemorrhage. After a 50-mL syringe (JMS Co., Ltd.) was filled with a viscoelastic composition specimen that had been temperature-regulated to 25°C, the syringe was connected to one end of an irrigator (BioShield irrigator (HZ-711133), US endoscopy), the other end of the irrigator was connected to a connector of an endoscope, and then the syringe was operated to continuously inject the viscoelastic composition specimen via a forceps channel into the plastic tube at an injection rate of about 0.5 to 5 mL/s and at the same time, the performance for securing the field of view was evaluated. The performance for securing the field of view was comprehensively evaluated from the viewpoint of securing a physical transparent space (whether a sufficient transparent space could be secured by pushing away blood (suppressing the flow or diffusion of blood) or not, when the specimen was injected) and maintaining transparency (whether transparency could be maintained or not, even when the injection of the specimen was stopped). When the injected specimen made possible observation inside the plastic tube (observation of the needle tip of the SURFRO indwelling needle), the specimen was concluded to be "acceptable", but when the observation inside the plastic tube was difficult, the specimen was concluded to be "unacceptable". For example, when observation of the inside of the plastic tube (observation of the needle tip of the SURFRO indwelling needle) was impossible, such as when it was necessary to inject a large amount of a specimen in order to secure a physical transparent space or when, after stopping injection of a specimen, blood rapidly invaded into the specimen and transparency was impaired or the like, the specimen was concluded to be "unacceptable". Furthermore, when a specimen was concluded to be "acceptable" in the performance for securing the field of view, the specimen was further evaluated in the visibility of the needle tip. Specifically, when the needle tip of the SURFRO indwelling needle was visible, the specimen was concluded to be "+" in visibility, and when the needle tip of the SURFRO indwelling needle was clearly visible, the specimen was concluded to be "++" in visibility.

(4) How to evaluate specimens in channel passability

In a laboratory set to room temperature of 23°C, a 50-mL syringe (JMS Co., Ltd.) was filled with a viscoelastic composition specimen that had been temperature-controlled to 25°C, a tip of the syringe was attached to a catheter tube (inner diameter: 3 mm, and length: 1,000 mm) simulating the inner diameter of a forceps opening of an endoscope, and the passability of the viscoelastic composition specimen was subjectively judged. When the specimen passed smoothly, the specimen was concluded to be "++"; when the passage was in a practically acceptable range with slight resistance, the specimen was concluded to be "+"; and when the resistance too much to use in practice or the resistance too much so that the specimen could not pass, the specimen was concluded to be "-".

**Test Examples: As to relations between viscoelastic properties (G' and tan $\delta$) of viscoelastic composition used for endoscope and securement of field of view of endoscope**

Viscoelastic composition specimens having different viscoelastic properties were prepared, the acceptability of the performance for securing the field of view was determined for each of the specimens, and the relations between the performance for securing the field of view and various viscoelastic properties were evaluated. The test results were as follows.

[Table 1]

| Raw material | Weight ratio | Concentration (wt%) | G' (Pa) | G" (Pa) | tan δ | Visibility of needle tip | Acceptability |
|---|---|---|---|---|---|---|---|
| XG/GG | 7/3 | 0.22 | 0.47 | 0.54 | 1.15 | - | Unacceptable |
| XG/GG | 7/3 | 0.24 | 0.60 | 0.64 | 1.06 | - | Unacceptable |
| XG/GG | 7/3 | 0.26 | 0.76 | 0.73 | 0.97 | + | Acceptable |
| XG/GG | 7/3 | 0.28 | 1.00 | 0.85 | 0.86 | + | Acceptable |
| XG/LBG | 2/8 | 0.057 | 1.03 | 0.37 | 0.36 | + | Acceptable |
| XG/LBG | 2/8 | 0.059 | 1.12 | 0.36 | 0.32 | + | Acceptable |
| XG/GG | 7/3 | 0.3 | 1.17 | 0.93 | 0.80 | + | Acceptable |
| XG/LBG | 2/8 | 0.061 | 1.28 | 0.35 | 0.27 | + | Acceptable |
| XG/LBG | 2/8 | 0.059 | 1.33 | 0.40 | 0.30 | ++ | Acceptable |
| XG/GG | 7/3 | 0.33 | 1.41 | 1.04 | 0.74 | ++ | Acceptable |
| XG/GG | 7/3 | 0.32 | 1.46 | 1.05 | 0.72 | ++ | Acceptable |
| XG/LBG | 2/8 | 0.063 | 1.67 | 0.42 | 0.25 | ++ | Acceptable |
| XG/GG | 7/3 | 0.34 | 1.72 | 1.15 | 0.67 | ++ | Acceptable |
| XG/GG | 7/3 | 0.36 | 1.79 | 1.22 | 0.69 | ++ | Acceptable |
| XG/GG | 7/3 | 0.40 | 2.18 | 1.38 | 0.63 | ++ | Acceptable |
| XG/LBG | 2/8 | 0.080 | 2.64 | 0.65 | 0.25 | ++ | Acceptable |
| XG/GG | 7/3 | 0.43 | 2.73 | 1.60 | 0.58 | ++ | Acceptable |
| XG/GG | 7/3 | 0.46 | 3.32 | 1.82 | 0.55 | ++ | Acceptable |
| XG/LBG | 2/8 | 0.1 | 3.99 | 0.34 | 0.08 | ++ | Acceptable |
| XG/LBG | 2/8 | 0.095 | 4.00 | 0.34 | 0.08 | ++ | Acceptable |
| XG/GG | 7/3 | 0.50 | 4.02 | 2.07 | 0.51 | ++ | Acceptable |
| XG/LBG | 2/8 | 0.11 | 4.30 | 0.39 | 0.09 | ++ | Acceptable |
| XG/LBG | 2/8 | 0.12 | 5.78 | 0.40 | 0.07 | ++ | Acceptable |
| XG/LBG | 2/8 | 0.13 | 6.95 | 0.44 | 0.06 | ++ | Acceptable |

From these results, it was demonstrated that irrespective of the raw materials of the viscoelastic composition themselves, all specimens having a shear storage modulus G' of 0.7 Pa or greater gave desired performance in securing the field of view. Further, it was demonstrated that even when the loss tangent of the specimen was higher than 0.6, the desired field of view could be secured as long as the shear storage modulus G' was 0.7 Pa or more,

**Test Examples: As to relations between viscoelastic properties (G' and tan δ) of viscoelastic composition used for endoscope and channel passability**

Viscoelastic composition specimens having different viscoelastic properties were prepared, the channel passability was determined for each of the specimens, and the relations between the channel passability and various viscoelastic properties were evaluated. The test results were as follows.

[Table 2]

| Raw material | Weight ratio | Concentration (wt%) | G' (Pa) | G" (Pa) | tanδ | Channel passability |
|---|---|---|---|---|---|---|
| XG/LBG | 5/5 | 0.08 | 3.26 | 0.34 | 0.11 | ++ |
| XG/LBG | 8/2 | 0.30 | 6.27 | 1.08 | 0.17 | + |
| XG/LBG | 6/4 | 0.20 | 10.78 | 0.77 | 0.07 | - |

From these results, it was demonstrated that viscoelastic compositions having lower shear storage modulus G' were preferable from the viewpoint of channel passability.

**Reference Signs List**

| | |
|---|---|
| 1 | Endoscope distal part |
| 2 | Hemorrhage site |
| 3, 3' | Blood |
| 3" | Turbid water |
| 4 | Alimentary canal |
| 5 | Viscoelastic composition |
| 11 | Dial |
| 12 | Scope connector unit |
| 13 | Tube |
| 14 | Connector |
| 15 | Forceps opening |
| 16 | Forceps lid |
| 17 | Endoscope flexible tube |

**Claims**

1. A viscoelastic composition for securing the field of view of an endoscope, the viscoelastic composition comprising a thickening substance and water and having a shear storage modulus G' of 0.7 Pa or more, wherein the viscoelastic composition is colorless and transparent, wherein the shear storage modulus G' is measured under the measurement conditions as follows: measurement temperature: 25.0°C, gap: 1.000 mm, stress: 1,000 mPa, and frequency: 0.5000 Hz, and the shear storage modulus G' is measured as a value obtained after 30 minutes from the initiation of measurement, and wherein the viscoelastic composition has a loss tangent of more than 0.6, wherein the loss tangent is measured under the measurement conditions as follows: measurement temperature: 25.0°C, gap: 1.000 mm, stress: 1,000 mPa, and frequency: 0.5000 Hz, and the loss tangent is measured as a value obtained after 30 minutes from the initiation of measurement, and wherein the thickening substance consists in a combination of a polysaccharide having, as the main chain, a structure in which glucose is glycoside-bonded and a polysaccharide having, as the main chain, a structure in which mannose is glycoside-bonded, and the weight ratio of the polysaccharide having, as the main chain, a structure in which glucose is glycoside-bonded to polysaccharide having, as the main chain, a structure in which mannose is glycoside-bonded is 5:95 to 90:10.

2. The viscoelastic composition according to claim 1, wherein the viscoelastic composition has a shear storage modulus G' of 7.0 Pa or less.

3. An endoscope comprising a viscoelastic composition for securing the field of view of an endoscope, the viscoelastic composition comprising a thickening substance and water and having a shear storage modulus G' of 0.7 Pa or more, wherein the viscoelastic composition is colorless and transparent, wherein the shear storage modulus G' is measured under the measurement conditions as follows: measurement temperature: 25.0°C, gap: 1.000 mm, stress: 1,000 mPa, and frequency: 0.5000 Hz, and the shear storage modulus G' is measured as a value obtained after 30 minutes from the initiation of measurement, and wherein the viscoelastic composition has a loss tangent of more than 0.6, wherein the loss tangent is measured under the measurement conditions as follows: measurement temperature: 25.0°C, gap: 1.000 mm, stress: 1,000 mPa, and frequency: 0.5000 Hz, and the loss tangent is measured as a value obtained after 30 minutes from the initiation of measurement and wherein the thickening substance consists in a combination of a polysaccharide having, as the main chain, a structure in which glucose is glycoside-bonded and a polysaccharide having, as the main chain, a structure in which mannose is glycoside-bonded, and the weight ratio of the polysaccharide having, as the main chain, a structure in which glucose is glycoside-bonded to polysaccharide having, as the main chain, a structure in which mannose is glycoside-bonded is 5:95 to 90:10.

4. The endoscope according to claim 3, wherein the viscoelastic composition has a shear storage modulus G' of 7.0 Pa or less.

**Patentansprüche**

1. Viskoelastische Zusammensetzung zur Sicherung des Sichtfelds eines Endoskops, wobei die viskoelastische Zusammensetzung eine Verdickungssubstanz und Wasser umfasst und einen Scherspeichermodul G' von 0,7 Pa oder mehr aufweist, wobei die viskoelastische Zusammensetzung farblos und transparent ist, wobei der Scherspeichermodul G' unter den folgenden Messbedingungen gemessen wird: Messtemperatur: 25,0 °C, Abstand: 1,000 mm, Belastung: 1000 mPa und Frequenz: 0,5000 Hz, und der Scherspeichermodul G' als ein Wert gemessen wird, der nach 30 Minuten ab Beginn der Messung erhalten wird, und wobei die viskoelastische Zusammensetzung eine Verlusttangente von mehr als 0,6 aufweist, wobei die Verlusttangente unter den folgenden Messbedingungen gemessen wird: Messtemperatur: 25,0 °C, Abstand: 1,000 mm, Belastung: 1000 mPa und Frequenz: 0,5000 Hz, und die Verlusttangente als ein Wert gemessen wird, der 30 Minuten nach Beginn der Messung erhalten wird, und wobei die Verdickungssubstanz aus einer Kombination eines Polysaccharids, das als Hauptkette eine Struktur aufweist, in der Glucose glycosidisch gebunden ist, und eines Polysaccharids, das als Hauptkette eine Struktur aufweist, in der Mannose glycosidisch gebunden ist, besteht und das Gewichtsverhältnis des Polysaccharids, das als Hauptkette eine Struktur aufweist, in der Glucose glycosidisch gebunden ist, zu dem Polysaccharid, die als Hauptkette eine Struktur aufweist, in der Mannose glycosidisch gebunden ist, 5:95 bis 90:10 beträgt.

2. Viskoelastische Zusammensetzung nach Anspruch 1, wobei die viskoelastische Zusammensetzung einen Scherspeichermodul G' von 7,0 Pa oder weniger aufweist.

3. Endoskop, das eine viskoelastische Zusammensetzung zur Sicherung des Sichtfelds eines Endoskops umfasst, wobei die viskoelastische Zusammensetzung eine Verdickungssubstanz und Wasser umfasst und einen Scherspeichermodul G' von 0,7 Pa oder mehr aufweist, wobei die viskoelastische Zusammensetzung farblos und transparent ist, wobei der Scherspeichermodul G' unter den folgenden Messbedingungen gemessen wird: Messtemperatur: 25,0 °C, Abstand: 1,000 mm, Belastung: 1000 mPa und Frequenz: 0,5000 Hz, und der Scherspeichermodul G' als ein Wert gemessen wird, der nach 30 Minuten ab Beginn der Messung erhalten wird, und wobei die viskoelastische Zusammensetzung eine Verlusttangente von mehr als 0,6 aufweist, wobei die Verlusttangente unter den folgenden Messbedingungen gemessen wird: Messtemperatur: 25,0 °C, Abstand: 1,000 mm, Belastung: 1000 mPa und Frequenz: 0,5000 Hz, und die Verlusttangente als ein Wert gemessen wird, der 30 Minuten nach Beginn der Messung erhalten wird, und wobei die Verdickungssubstanz aus einer Kombination eines Polysaccharids, das als Hauptkette eine Struktur aufweist, in der Glucose glycosidisch gebunden ist, und eines Polysaccharids, das als Hauptkette eine Struktur aufweist, in der Mannose glycosidisch gebunden ist, besteht und das Gewichtsverhältnis des Polysaccharids, das als Hauptkette eine Struktur aufweist, in der Glucose glycosidisch gebunden ist, zu dem Polysaccharid, die als Hauptkette eine Struktur aufweist, in der Mannose glycosidisch gebunden ist, 5:95 bis 90:10 beträgt.

4. Endoskop nach Anspruch 3, wobei die viskoelastische Zusammensetzung einen Scherspeichermodul G' von 7,0 Pa oder weniger aufweist.

**Revendications**

1. Composition viscoélastique de protection du champ de vision d'un endoscope, la composition viscoélastique comportant une substance épaississante et de l'eau et présentant un module de stockage au cisaillement G' de 0,7 Pa ou plus, dans laquelle la composition viscoélastique est incolore et transparente, dans laquelle le module de stockage au cisaillement G' est mesuré dans les conditions de mesure suivantes : température de mesure : 25,0 °C, écart : 1,000 mm, contrainte : 1000 mPa, et fréquence : 0,5000 Hz, et le module de stockage au cisaillement G' est mesuré par une valeur obtenue après 30 minutes à compter du début de la mesure, et dans laquelle la composition viscoélastique présente une tangente de perte supérieure à 0,6, dans laquelle la tangente de perte est mesurée dans les conditions de mesure suivantes : température de mesure : 25,0 °C, écart : 1,000 mm, contrainte : 1000 mPa, et fréquence : 0,5000 Hz, et la tangente de perte est mesurée par une valeur obtenue après 30 minutes à compter du début de la mesure, et dans laquelle la substance épaississante consiste en une combinaison d'un polysaccharide présentant, en tant que chaîne principale, une structure dans laquelle le glucose est lié par liaison glycosidique et d'un polysaccharide présentant, en tant que chaîne principale, une structure dans laquelle le mannose est lié par liaison glycosidique, et le rapport pondéral du polysaccharide présentant, en tant que chaîne principale, une structure dans laquelle le glucose est lié par liaison glycosidique sur le polysaccharide présentant, en tant que chaîne principale, une structure dans laquelle le mannose est lié par liaison glycosidique est de 5:95 à 90:10.

2. Composition viscoélastique selon la revendication 1, dans laquelle la composition viscoélastique présente un module de stockage au cisaillement G' de 7,0 Pa ou moins.

3. Endoscope comportant une composition viscoélastique de protection du champ de vision d'un endoscope, la composition viscoélastique comportant une substance épaississante et de l'eau et présentant un module de stockage au cisaillement G' de 0,7 Pa ou plus, dans lequel la composition viscoélastique est incolore et transparente, dans lequel le module de stockage au cisaillement G' est mesuré dans les conditions de mesure suivantes : température de mesure : 25,0 °C, écart : 1,000 mm, contrainte : 1000 mPa, et fréquence : 0,5000 Hz, et le module de stockage au cisaillement G' est mesuré par une valeur obtenue après 30 minutes à compter du début de la mesure, et dans laquelle la composition viscoélastique présente une tangente de perte supérieure à 0,6, dans laquelle la tangente de perte est mesurée dans les conditions de mesure suivantes : température de mesure : 25,0 °C, écart : 1,000 mm, contrainte : 1000 mPa, et fréquence : 0,5000 Hz, et la tangente de perte est mesurée par une valeur obtenue après 30 minutes à compter du début de la mesure, et dans lequel la substance épaississante consiste en une combinaison d'un polysaccharide présentant, en tant que chaîne principale, une structure dans laquelle le glucose est lié par liaison glycosidique et d'un polysaccharide présentant, en tant que chaîne principale, une structure dans laquelle le mannose est lié par liaison glycosidique, et le rapport pondéral du polysaccharide présentant, en tant que chaîne principale, une structure dans laquelle le glucose est lié par liaison glycosidique sur le polysaccharide présentant, en tant que chaîne principale, une structure dans laquelle le mannose est lié par liaison glycosidique est de 5:95 à 90:10.

4. Endoscope selon la revendication 3, dans lequel la composition viscoélastique présente un module de stockage au cisaillement G' de 7,0 Pa ou moins.

[Figure 1]

[Figure 2A]

[Figure 2B]

[Figure 3]

[Figure 4A]

[Figure 4B]

[Figure 5]

## REFERENCES CITED IN THE DESCRIPTION

### Patent documents cited in the description

- WO 2017057504 A

- JP 2014155677 A

### Non-patent literature cited in the description

- **YANO TOMONORI**. Gel immersion endoscopy: New method for securing field of view in urgent endoscopy. *Progress of Digestive Endoscopy*, 01 June 2015, vol. 87, s85

- **YANO T**. Gel immersion endoscopy: a novel method to secure the visual field during endoscopy in bleeding patients (with videos). *Gastrointestinal Endoscopy*, April 2016, vol. 83 (4), 809-811